# EUROPEAN PATENT SPECIFICATION

(11) **EP 0 576 743 B1**
(45) Date of publication and mention of the grant of the patent: **11.12.1996**
(21) Application number: 92305126.2
(22) Date of filing: 04.06.1992
(51) Int. Cl.: C12Q 1/68

(54) **Nucleic acid probes for the detection of mycoplasma pneumoniae**
Nukleinsäure Sonden für den Nachweis von Mycoplasma Pneumoniae
Sondes d'acides nucléiques pour la détection de mycoplasma pneumoniae

(43) Date of publication of application: 05.01.1994
(73) Proprietor: AMOCO CORPORATION, Chicago Illinois 60601 (US)
(72) Inventor: Weisburg, William Greene, Milford, MA 01757 (US); Pelletier, Dale Adam, Brighton, MA 02135 (US)
(74) Representative: Dowden, Marina

(56) References cited:
- EP-A- 0 250 662
- EP-A- 0 305 145
- WO-A-88/03957
- Journal for Genetic Microbiology, vol.133, July 1987, Colchester GB pages 1969-1974, U.B.Göbel et al. "Oligonucleotide probes complementary to variable regions of ribosomal RNA discriminate between Mycoplasma spevies"
- Journal of Clinical Micribiology, vol.25, no.4, April 1987, Washington US, pages 726-728, H.C.Hyman et al. "DNA probes for detection and identification of M.pneuminiae and M.genitalium"
- NUCLEIC AC.RES. vol.19,no 21,1991,p.6027-6031
- JOURNAL OF BACTERIOL.vol 171.no12,1989 p.6455-6467

## Description

### Field of the Invention

This invention relates to the detection of Mycoplasma pneumoniae, the causative agent of human primary atypical pneumonia. More specifically, it provides nucleic acid compositions and methods for their use for the specific detection of Mycoplasma pneumoniae in clinical and other samples. The nucleic acid compositions feature nucleotide sequences, from 10 to 250 nucleotides, which are capable of hybridizing preferentially to the rRNA and rDNA of Mycoplasma pneumoniae.

### Background of the Invention

Mycoplasmas are small wall-less bacteria, primarily isolated from animal sources including humans. There are over 70 members of the genus Mycoplasma, and several related genera which are also characterized by small wall-less bacteria; these are Spiroplasma, Acholeplasma, Ureaplasma, Anaeroplasma, and Asteroleplasma. Only a handful of the species within these genera have been found associated with humans--some presumed to be "normal flora", others suspected of being pathogenic, and only one species known to be an important cause of human morbidity, whenever it is isolated. Mycoplasma pneumoniae is an important cause of primary atypical pneumonia and several nonrespiratory complications. The indication of its presence always provides clinically relevant information.

Other pathogenic mycoplasma organisms comprise Mycoplasma fermentans, Mycoplasma hominis, Ureaplasma urealyticum and Mycoplasma genitalium. Nucleic acid compositions and methods for the detection of these organisms are the subject of two concurrently filed applications USSN 07/673,661 and USSN 07/673,687 entitled "Nucleic Acid Probes For The Detection of Genital Mycoplasmas" and "Nucleic Acid Probes For The Detection Of Mycoplasma Fermentans Or The Aids-Associated Virus-Like Infectious Agent." At least one inventor is common to both of these applications and the present application.

The mycoplasmas, such as Mycoplasma pneumoniae, are fastidious organisms, requiring complex culture media containing peptone, yeast extract, expensive animal sera, and sterol. Growth is relatively slow and reaches low cell densities compared to most bacteria. In addition, atmospheric conditions for cell growth require the addition of carbon dioxide. For these reasons, many clinical laboratories are unable to perform culture isolation of M. pneumoniae, and consequently are left with no real ability to diagnose the presence of this important pathogenic bacterium. Given that mycoplasmas lack cell walls, antibiotics that target the bacterial cell wall, such as penicillin, have no anti-mycoplasma activity. Consequently, it is of importance for a physician to make a diagnosis of atypical pneumonia and prescribe the appropriate antibiotic.

Several investigators have discussed the similarity of Mycoplasma pneumoniae to Mycoplasma genitalium both serologically (Lind, et al., J. Clinical Microbiol., vol. 20, 1984) and within DNA sequences including the rRNA operons (Yogev and Razin, Int. Jnl. System. Bacteriol., vol. 36, 1986). Weisburg, et al. discuss the various other evolutionary relatives of M. pneumoniae (Jnl. of Bacteriol., vol. 171, 1989). In particular, this reference describes the GenBank accession numbers for the nucleotide sequences of small subunit ribosomal RNA sequences of about 50 species of mycoplasmas.

M. genitalium may possibly have a role in respiratory infection either in co-culture with M. pneumoniae or in the absence of M. pneumoniae (Tully, Clinical Micro. Newsletter, vol. 11, 1989). Mycoplasma genitalium may be responsible for some fraction of clinical atypical pneumonia.

Peterson et al. (Nuc. Acids Res., 21: 6027-6031 (1991)) describes the use of a random sequencing approach to place markers on the genomic map of Mycoplasma genitalium.

While Kohne et al. (Biophysical Journal 8:1104-1118, 1968) discuss one method for preparing probes to rRNA sequences, they do not provide the teaching necessary to make probes to detect Mycoplasma pneumoniae or M. pneumoniae in combination with M. genitalium.

Pace and Campbell (Journal of Bacteriology 107:543-547, 1971) discuss the homology of ribosomal ribonucleic acids from diverse bacterial species and a hybridization method for quantitating such homology levels. Similarly, Sogin, Sogin and Woese (Journal of Molecular Evolution 1:173-184, 1972) discuss the theoretical and practical aspects of using primary structural characterization of different ribosomal RNA molecules for evaluating phylogenetic relationships. Fox, Pechman and Woese (International Journal of Systematic Bacteriology 27:44-57, 1977) discuss the comparative cataloging of 16S ribosomal RNAs as an approach to prokaryotic systematics.

Hogan, et al. (International Patent Application, Publication Number WO 88/03957) describe five putative M. pneumoniae specific probes, but they were not tested in a manner which allowed evaluation of M. genitalium cross-reactivity by rigorous criteria. Their mixture of four probes reacted 10 times more strongly with M. genitalium than with the related species, M. gallisepticum. None of their probes target the 23S rRNA molecule. Probes disclosed appear to be unable to distinguish a known homologous sequence from M. genitalium.

Zivin and Monahan, European Patent application publication number 0305145A2, describe several probes which were designed based on the determination of 307 bases of the 5' end of M. pneumoniae. They claim anecdotally to exclude M. genitalium with some of their probes.

Gobel, et al. discuss probes for Mycoplasmas including M. pneumoniae (Gobel, et.al., Israel Jnl. of Med. Sci. vol. 23, 1987) but fail to teach what the structure of these probes is. Further, in an EP patent application number, EP0250662A1, Gobel et al. suggest probes to M. pneumoniae; however, such probes may lack the sensitivity or specificity for clinical applications and are not suitable for solution hybridization formats.

Rogers, et al. discuss sequences of 5S rRNA of mycoplasmas (Rogers et al., Proc. Natl. Acad. Sci. USA, vol. 82, 1985). Rogers, et al. do not suggest any sequences which would be useful to facilitate detection of these organisms and do not discuss 16S or 23S rRNA sequences. Woese, et al. discuss 16S rRNA oligonucleotide catalogs of selected mycoplasma species, but do not discuss probes or Mycoplasma pneumoniae.

Bernet, et al. discuss polymerase chain reaction detection of Mycoplasma pneumoniae (Bernet, et al., Jnl. Clin. Micro., vol. 27, 1989), but not directed toward rRNA or rDNA sequences. Hyman, et al. also discuss polymerase chain reaction detection of Mycoplasma pneumoniae and Mycoplasma genitalium (Hyman, et al., Jnl. Clin. Micro., vol. 25, 1987), but fail to discuss the utility of rRNA or rDNA sequences.

Gobel, et al. discuss mycoplasma rRNA probes in a publication, (Gobel, et al., Jnl. Genl. Micro., vol 133, 1987). However, Gobel, et al. do not recognize the significance of discrimination of probes toward other mycoplasma species, particularly Mycoplasma genitalium.

Ribosomes are of profound importance to all organisms because they serve as the only known means of translating genetic information into cellular proteins, the main structural and catalytic elements of life. A clear manifestation of this importance is the observation that all cells have ribosomes.

Bacterial ribosomes contain three distinct RNA molecules which, at least in Escherichia coli, are referred to as 5S, 16S and 23S rRNAs. In eukaryotic organisms, there are four distinct rRNA species, generally referred to as 5S, 18S, 28S, and 5.8S. These names historically are related to the size of the RNA molecules, as determined by their sedimentation rate. In actuality, however, ribosomal RNA molecules vary substantially in size between organisms. Nonetheless, 5S, 16S, and 23S rRNA are commonly used as generic names for the homologous RNA molecules in any bacterium, including the mycoplasmas, and this convention will be continued herein.

As used herein, probe(s) refer to synthetic or biologically produced nucleic acids (DNA or RNA) which, by design or selection, contain specific nucleotide sequences that allow them to hybridize under hybridization conditions, preferentially to target nucleic acid sequences. The term "preferentially" is used in a relative sense; one hybridization reaction product is more stable than another under identical conditions.

In addition to their hybridization properties, probes also may contain certain constituents that pertain to their proper or optimal functioning under particular assay conditions. For example, probes may be modified to improve their resistance to nuclease degradation (e.g. by end capping), to carry detection ligands (e.g. fluorescein, biotin, and avidin), to facilitate direct or indirect detection (³²P, and fluorescent and chemiluminescent agents) or to facilitate their capture onto a solid support (e.g., homopolymer "tails"). Such modifications are elaborations on the basic probe function which is its ability to usefully discriminate between target and non-target organisms in a hybridization assay.

Hybridization traditionally is understood as the process by which two partially or completely complementary strands of nucleic acid are allowed to come together in an antiparallel fashion (one oriented 5' to 3', the other 3' to 5') to form a double-stranded nucleic acid with specific and stable hydrogen bonds, following explicit rules pertaining to which nucleic acid bases may pair with one another. The high specificity of probes relies on the low statistical probability of unique sequences occurring at random as dictated by the multiplicative product of their individual probabilities. Normal hybridization conditions for nucleic acid of approximately 10 to 250 nucleotides would include a temperature of approximately 60°C in the presence of 1.08 M sodium chloride, 60 mM sodium phosphate and 6 mM ethylenediamine tetraacetic acid (pH 7.4).

Hybridization conditions are easily modified to suit nucleic acid of differing sequences. Factors which may influence the hybridization conditions for a particular nucleic acid composition are base composition of the probe/target duplex, as well as the level and geometry of mispairing between the two nucleic acids.

Reaction parameters which are commonly adjusted are the concentration and type of ionic species present in the hybridization solution, the types and concentrations of denaturing agents present, and the temperature of hybridization. Generally, as hybridization conditions become more stringent, or less favorable for hybridization, longer probes are required to form stable hybrids.

### Summary of the Invention

The present invention features nucleic acid compositions and composition sets and methods for the specific detection or identification of pathogenic mycoplasma bacteria associated with human primary atypical pneumonia. One embodiment of the present invention features, as a composition of matter, a nucleic acid having approximately 10 to 250 nucleotides capable of hybridizing to rRNA or DNA encoding rRNA (hereinafter referred to as rDNA) of pathogenic mycoplasma bacteria associated with human primary atypical pneumonia in preference to rRNA or rDNA of nonmycoplasma bacteria and humans. The nucleic acid compositions are useful for detecting Mycoplasma peumoniae, and/or Mycoplasma genitalium.

Embodiments of the present invention feature nucleic acids capable of hybridizing to 16S rRNA or DNA encoding 16S rRNA of Mycoclasma pneumoniae and 23S rRNA or DNA encoding 23S rRNA of Mycoplasma pneumoniae. One embodiment of the present invention features a nucleic acid capable of hybridizing to 16S rRNA or DNA encoding 16S rRNA, or 23S rRNA or DNA encoding 23S rRNA of both Mycoplasma pneumoniae and Mycoplasma genitalium.

According to one embodiment, therefore, the present invention is directed to a nucleic acid probe that hybridizes stably to the 16S rRNA or DNA encoding 16S rRNA of *Mycoplasma pneumoniae* and does not hybridize stably to the 16S rRNA or DNA encoding 16S rRNA of non-*Mycoplasma* bacteria and humans under identical hybridization conditions, the probe having a sequence complementary to or homologous with at least a portion of the sequence of Probe 2167 (CCAATTTGCATTAGCAGTCTCGCTAGACAATGTAACT).

In addition, the invention is directed to a nucleic acid probe that hybridizes stably to the 23S rRNA or DNA encoding 23S rRNA of *Mycoplasma pneumoniae* and does not hybridize stably to the 23S rRNA or DNA encoding 23S rRNA of non-*Mycoplasma* bacteria and humans under identical hybridization conditions, the probe having a sequence complementary to or homologous with at least a portion of the sequence of any one of Probe 2295 (TCACCACGTATTGCTTTAATTGACTATTTATTCATCAAT), Probe 2289 (CAACCCCTATCTAATGATAAGTTTGGCCTGT), Probe 2296 (TTCTATCGTTTTCAAGTC CACATTGCAAGCCCTAC), Probe 2298 (TGGTTACAGCTAGATTAACCCTAGAAGCTTTT CT), probe 2299 (CCTATTCTCTACATGATAATGTCCTGATCAATATT), Probe 2300 (TAGTATTCCACCTTTCGCATCAACAAGTCCTAGCGAACT), or Probe 2294 (TAGAAGC AACACTCTTCAATCTTCCTATCGGGCACAAT).

According to a second embodiment, the invention is directed to a nucleic acid probe that hybridizes stably to the 16S rRNA or DNA encoding 16S rRNA of *Mycoplasma pneumoniae* and *Mycoplasma genitalium* and does not hybridize stably to the 16S rRNA or DNA encoding 16S rRNA of non-Mycoplasma bacteria and humans under identical hybridization conditions, the probe having a sequence coomplementary to or homologous with at least a portion of the sequence of Probe 2202 (CTTTGATTCATGCGAACCAAAGTTCTTATGCGGTATTAGC TAGTCTTTC) , Probe 2166 (CTCCCTACCACACTCTAGATTAATAGTTTCCAATGC), probe 2225 (CTTTTCAGATTTGCTCACTTTTACAAGTTGGCTACTGT), or Probe 2230 (CTTTAATTCATGCGAACTAAAGTTCTTATGCGGTATTAGCTAGTTTTC).

In addition, the invention is further directed to a nucleic acid probe that hybridizes stably to the 23S rRNA or DNA encoding 23S rRNA of *Mycoplasma pneumoniae* and *Mycoplasma genitalium* and does not hybridize stably to the 23S rRNA or DNA encoding 23S rRNA of non-Mycoplasma bacteria and humans under identical hybridization conditions, the probe having a sequence complementary to or homologous with at least a portion of the sequence of Probe 2297 (GCTATCACCCTTTTGCGCGCTGCTTTCCAAT).

Those nucleic acid compositions of the present invention that are capable of hybridizing to rRNA or rDNA of both Mycoplasma pneumoniae and Mycoplasma genitalium have utility in two-probe systems in which one of the two probes is capable of distinguishing between the two organisms. Therefore, according to a further embodiment of the invention, there is provided a set of nucleic acid probes comprising at least two nucleic acid probes, each probe hybridizing stably to the 16S rRNA or DNA encoding 16S rRNA of *Mycoplasma pneumoniae* and not hybridizing stably to the 16S rRNA or DNA encoding 16S rRNA of non-Mycoplasma bacteria and humans under identical hybridization conditions, and each probe having a different base sequence composition, wherein a first nucleic acid probe has a sequence complementary to or homologous with at least a portion of the sequence of any one of Probe 2167 (CCAATTTGCATTAGCAGTCTCGCTAGACAATGTAACT), Probe 2166 (CTCCCTACCACACTCTAGATTAATAGTTTCCAATGC), Probe 2202 (CTTTGATTCATGCGAACCAAAGTTCTTATGCGGTATTAGCTAGTCTTTC) or Probe 2230 (CTTTAATTCATGCGAACTAAAGTTCTTATGCGGTATTAGCTAGTTTTC), and a second nucleic acid probe has a sequence complementary to or homologous with at least a portion of the sequence of any one of Probe 2167, Probe 2162 (CGCCTCTAAAGTATTACTACTTTCGATCGACTTGCATGT), Probe 2166, Probe 2196 (CTTTACAGATTTGCTCACTTTTACAAGCTGGCGACTGT), Probe 2192 (CCCTCATCAAATAACGAACCCTTGCAGGTCCT), Probe 2219 (CCTCCATTATGTTTCCATAACTTTGCCAAGGATGT), Probe 2201 (CAAAATGGTACAGTC AAACTCTAGCCATTACCTGCTAAAGTCATTC), Probe 2224 (CACTACCGAGGGGATCGCCCCGACAGCTAGTATCTATCGT), Probe 2195 (GCTCACTTTTACAAGCTGGCGACTGTTTG TATTGGCC) or Probe 2225 (CTTTTCAGATTTGCTCACTTTTACAAGTTGGCTACTGT).

In addition, there is further provided a set of nucleic acid probes comprising at least two nucleic acid probes, each probe hybridizing stably to the 23S rRNA or DNA encoding 23S rRNA of *Mycoplasma pneumoniae* and not hybridizing stably to the 23S rRNA or DNA encoding 23S rRNA of non-Mycoplasma bacteria and humans under identical hybridization conditions, and each probe having a different base sequence composition and having a sequence complementary to or homologous with at least a portion of the sequence of any one of Probe 2295, Probe 2289, Probe 2296, Probe 2298, ?robe 2299, Probe 2300, Probe 2294, or Probe 2297.

Preferably, the set of nucleic acid probes is selected from the group of sets consisting of,
Probe 2196 and Probe 2167; Probe 2162 and Probe 2202;
Probe 2224 and Probe 2166; Probe 2162 and Probe 2230;
Probe 2167 and Probe 2219; Probe 2192 and Probe 2202;
Probe 2296 and Probe 2297; and, Probe 2299 and Probe 2300.

A further embodiment of the present invention features a method for detecting the presence of one or more mycoplasmas which consist essentially of Mycoplasma pneumoniae and Mycoplasma genitalium in a sample. The method includes the steps of contacting a sample with at least one nucleic acid probe having a sequence complementary to or homologous with at least a portion of the sequence of probes 2167, 2295, 2289, 2296, 2298, 2299, 2300, 2294, 2202, 2166, 2225, 2230, or 2297. The method includes imposing hybridization conditions on the sample which conditions allow the nucleic acid probe to hybridize stably to the rRNA or DNA encoding rRNA of either one or both of *Mycoplasma pneumoniae* and *Mycoplasma genitalium,* if present in the sample, to form nucleic acid complexes, under conditions which do not allow said probe to form stable hybridized nucleic acid complexes with non-Mycoplasma bacteria. The method also involves detecting the nucleic acid complexes as an indication of the presence of either one or both of *Mycoplasma pneumoniae* and *Mycoplasma genitalium* in the sample.

Preferably, the nucleic acid of the contacting step is complementary to or homologous with at least 90% of a sequence comprising any ten consecutive nucleotides selected from the group of sequences defined by probes 2295, 2289, 2296, 2298, 2299, 2300, 2294, 2167, 2297, 2202, 2219, and 2230.

One embodiment of the present invention features a contacting step utilizing two nucleic acid compositions. Each nucleic acid has 10 to 250 base sequences capable of hybridizing preferentially to mycoplasma rRNA and rDNA and each having a different base composition. The nucleic acids are complementary to or homologous with at least 90% of a sequence comprising any 10 consecutive nucleotides selected from the group of sequences defined by probes and selected from the group of sets consisting of,
Probe 2196 + Probe 2167, Probe 2162 + Probe 2202
Probe 2224 + Probe 2166, Probe 2162 + Probe 2230
Probe 2167 + Probe 2219, Probe 2192 + Probe 2202
Probe 2296 + Probe 2297, Probe 2299 + Probe 2300.

A further embodiment of the present invention features a kit for detecting pathogenic mycoplasma bacterium associated with human primary atypical pneumonia. The kit has a nucleic acid probe having 10 to 250 base sequences, capable of hybridizing to rRNA or rDNA encoding rRNA of pathogenic mycoplasma bacteria in preference to rRNA and rDNA encoding rRNA of nonmycoplasma and humans. The nucleic acid probe of said kit comprises a nucleic probe described hereinbefore or a set of nucleic acid probes according to the invention, described hereinbefore.

Typically, kits are comprised of reagents, compositions, instructions, disposable hardware and suitable packaging to allow marketing a convenient assembly.

The nucleic acid compositions, kits, and methods of the present invention provide the basis for development of valuable nucleic acid hybridization assays for the specific detection of atypical pneumonia or its etiological agent. The type of samples which may be encountered include sputum, throat swabs, blood, urine, cerebrospinal fluid, skin, biopsy, saliva, synovial fluid, bronchial wash, bronchial lavage, or other tissue or fluid samples from human patients or veterinary subjects.

The discovery that probes could be generated with the extraordinary inclusivity and exclusivity characteristics of the present invention with respect to the detection of a panel of M. pneumoniae and M. genitalium isolates, without necessarily incurring cross-reactivity between these species, was unpredictable and unexpected.

### Brief Description of the Tables and Figures

Further understanding of the principles and aspects of the present invention may be made by reference to the tables and figures:
Tables 1 and 2 describe the structure of the target regions and display the physical structure of the probes.
Table 3 displays the hybridization behavior of the the probes toward a panel of clinically and environmentally representative mycoplasma species.

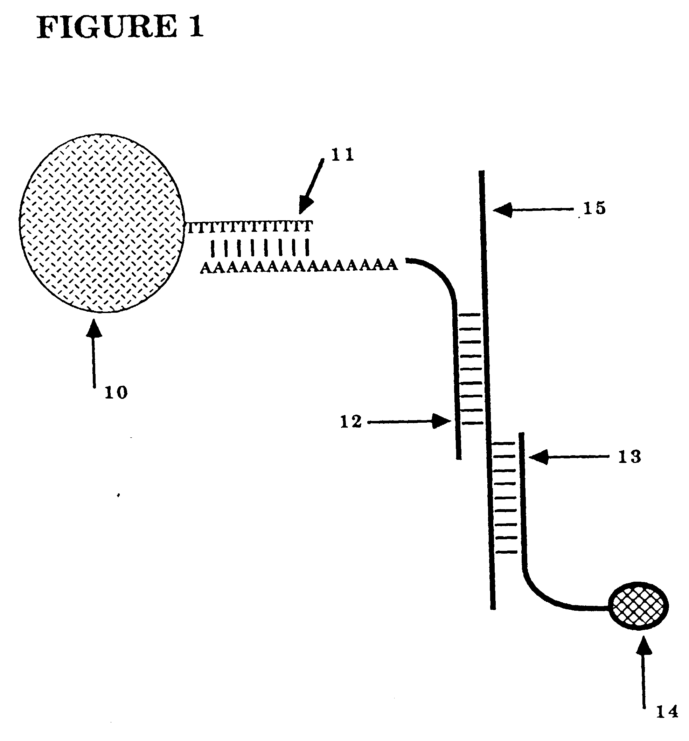

Figure 1 is a schematic representation of a dual probe capture/detector assay.

### Detailed Description of the Invention and Best Mode

### Probe Development Strategy

The first step taken in the development of nucleic acid compositions of the present invention involved identification of regions of the 16S rRNA which potentially could serve as target sites for specific probes with the desired sensitivity. As stated, this desired sensitivity included finding probe targets unique to Mycoplasma pneumoniae, and finding probe targets common to both M. pneumoniae and M. genitalium. This entailed finding sites which are:
(1) different between M. pneumoniae and M. genitalium, and
(2) substantially the same within the two species' sequences, but different within the next closest evolutionary neighbors' sequences.

For this analysis, precise alignments of mycoplasma 16S and 23S rRNA sequences were developed. The essentially complete 16S rRNA and 23S rRNA sequences of both M. pneumoniae and M. genitalium were determined as part of this effort. Such nucleotide sequences were determined by standard laboratory protocols well known to those skilled in the art of probe design. The 16S and 23S rDNAs were cloned into plasmid vectors from products produced by enzymatic amplification. The Mycoplasma pneumonia and M. genitallum sequences were aligned with homologous sequences of other mycoplasma and nonmycoplasma ribosomal RNA sequences. In addition to specifically addressing the close clinical and evolutionary relationship of M. pneumoniae to M*.* genitalium, the following additional 16S rRNA sequences were known and evaluated: M. agalactiae, M. arginini, M. arthritidis, M. bovigenitalium, M. californicum, M. capricolum, M. ellychniae, M. melaleucum, M. fermentans, M. gallisepticum, M. hominis, M. hyopneumoniae, M. hyorhinis, M. iowae, M. lipophilum, M. mobile, M. muris, M. mycoides, M. neurolyticum, M. orale, M. pirum, M. pneumoniae, M. pulmonis, M. putrefaciens, M. salivarium, M**.** sualvi, ten Spiroplasma species' sequences, four Acholeplasma species' sequences, four Anaeroplasma species' sequences, one Asteroleplasma sequence, and Ureaplasma urealyticum 16S rRNA sequence. (GenBank accession numbers shown in Weisburg et.al., Jnl. of Bacteriology, vol 171. 1989).

Twenty probes--designed, synthesized, and tested--are described by sequences in Tables 1 and 2. Nine probes specifically discriminate between M. pneumoniae and M. genitalium, eleven probes are uniquely inclusive for the pair of species. Probes which hybridize to both of the species have at least two benefits:
(1) as mentioned above, M. genitalium may be the cause of significant morbidity which is indistinguishable from that caused by M. pneumoniae, and
(2) probes can be incorporated into dual probe assays in which only one of the probes requires the desired specificity.

### Description of the Probes

The probe selection strategy yielded twenty probes useful for hybridization to the etiological agent of primary atypical pneumonia in samples.

Table 1 displays the precise alignment of the probe regions for both Mycoplasma pneumoniae and Mycoplasma genitalium along with the probe sequences designed from the aligned targets.

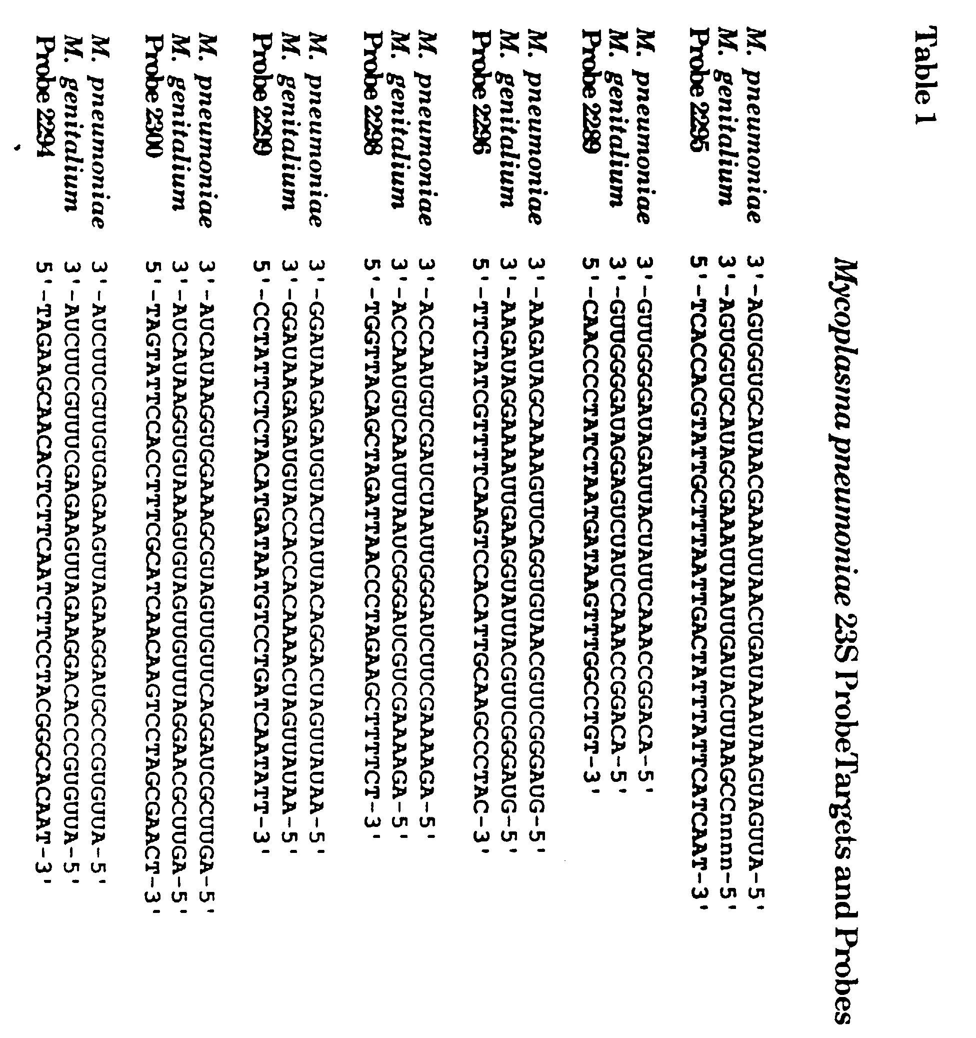

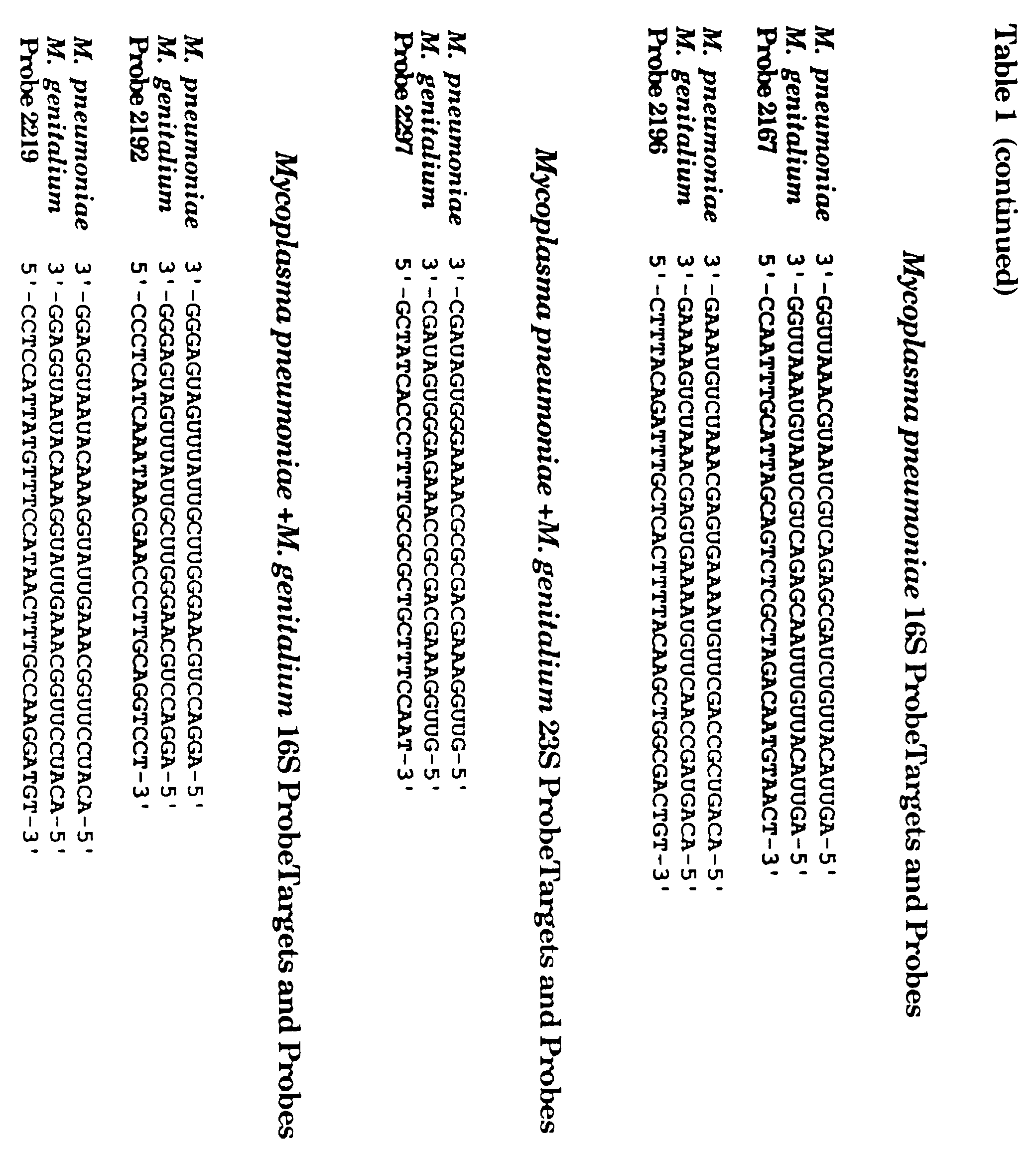

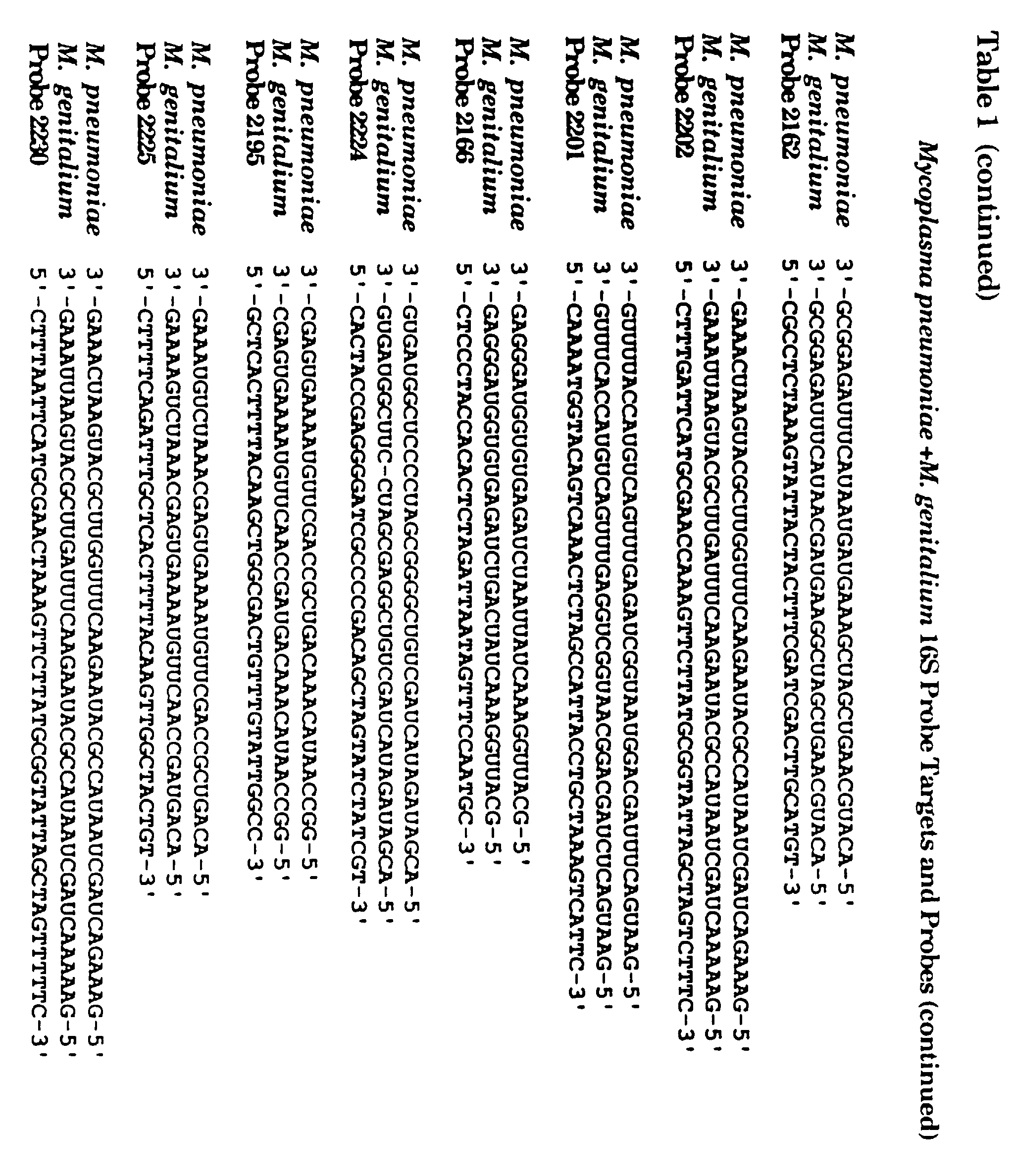

Table 2 summarizes specific preferred nucleic acid compositions including length and G+C content.

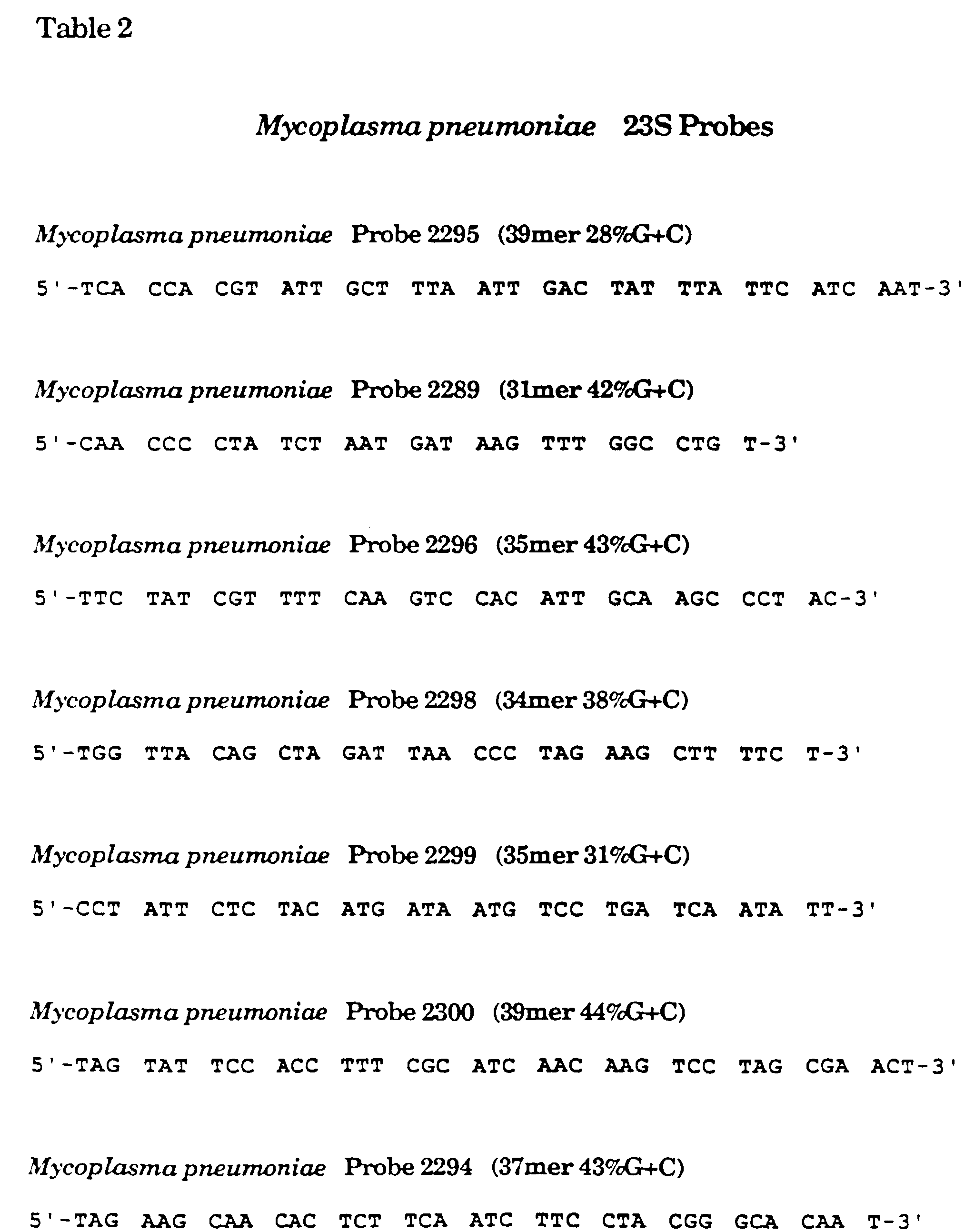

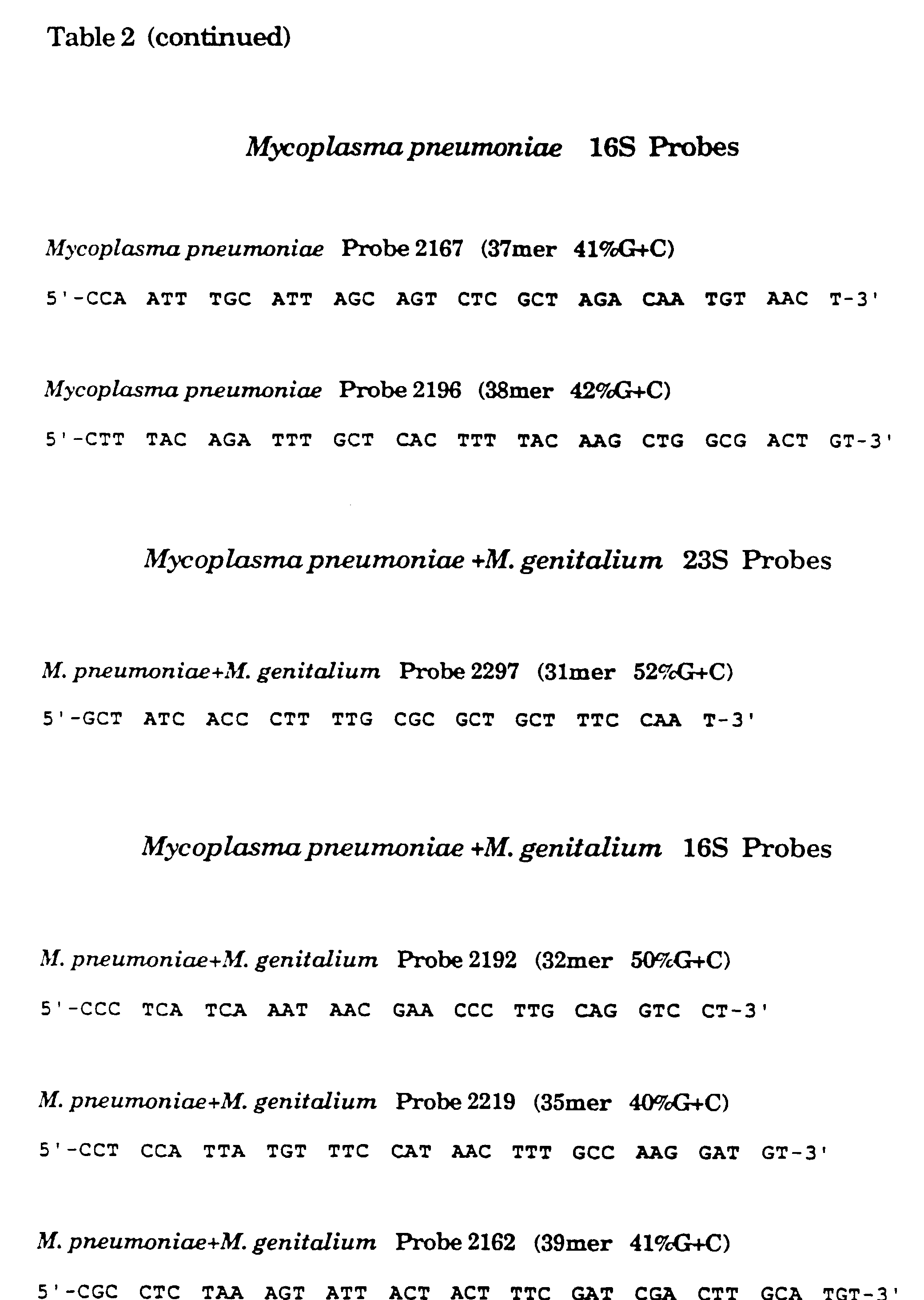

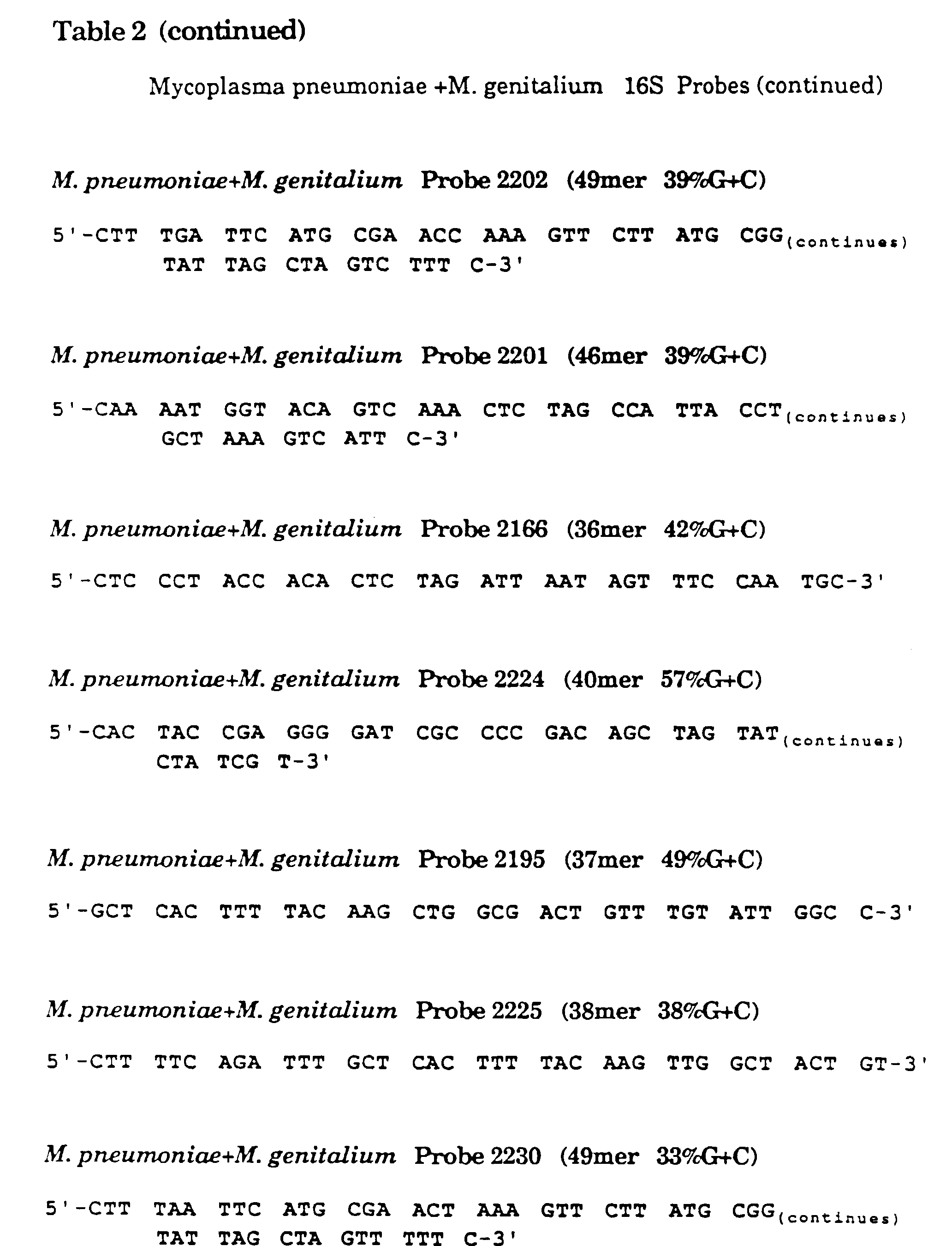

The nucleic acid compositions of the invention should not be construed as restricted to the specific nucleotides identified with the named probes. Optimal probe length will be a function of the stringency of the hybridization conditions chosen. The sequences identified with the named probes are capable of some alteration and modification. Indeed, individuals skilled in the art will readily make adjustments in probe length and modified nucleotides to serve their specific assay and detection needs. For example, the length of these particular oligonucleotides was optimized for use in the dot blot assay and sandwich formats. In considering sets comprised of more than one probe, it is desirable that all probes behave in a compatible manner. Thus, the exact length of a particular probe will to a certain extent reflect its specific intended use.

The nucleic acid compositions of the present invention comprise sequences which can be employed as oligonucleotide probes, or could be incorporated into larger polynucleotides of either ribonucleic acid or deoxyribonucleic acid. Sequences complementary to the probes described herein can be used as probes to rRNA genes. The preferred probes or their complements can be employed as chain elongation initiators for polymerase chain reaction, sequencing or other applications.

### Examples

### Example 1. Dot-Blot Analysis of Probe Hybridization Behavior

Dot-blot analysis, involves immobilizing a nucleic acid or a population of nucleic acids on a filter such as nitrocellulose, nylon, or other derivatized membranes which can readily be obtained commercially, specifically for this purpose. Either DNA or RNA can be easily immobilized on such a filter and subsequently can be probed or tested for hybridization under any of a variety of conditions i.e., stringencies) with nucleic acid compositions or probes of interest. Nucleic acids which have sequences with greater complementarity to a target will form hybridization reaction products which are more stable than probes containing less complementarity. Indeed, the added stability can be exploited to allow nucleic acids having sequences complementary to a target to bind preferentially to target without substantial hybridization to nontarget nucleic acids.

Probes of the present invention were tested in a dot-blot format. One hundred nanograms of RNA, purified by phenol extraction and centrifugation through cesium trifluoroacetate gradients, was denatured and spotted on a nylon membrane. Probes were isotopically labelled with the addition of a ³²Phosphorous moiety to the 5' end of the oligonucleotide. Hybridization of probes occurred, at a temperature of 60°C in the presence of 1.08 M sodium chloride, 60 mM sodium phosphate, and 6 mM ethylenediamine tetraacetic acid, pH 7.4. Unhybridized probe was removed by washing at a salt concentration one-third of the hybridization condition. The filters were exposed to X-ray film and the intensity of hybridization signals was evaluated after three hours of exposure.

The experimental specificity of the preferred probes, as described in Example 1 is summarized in Table 2 shown hereinbefore and Table 3 shown below.

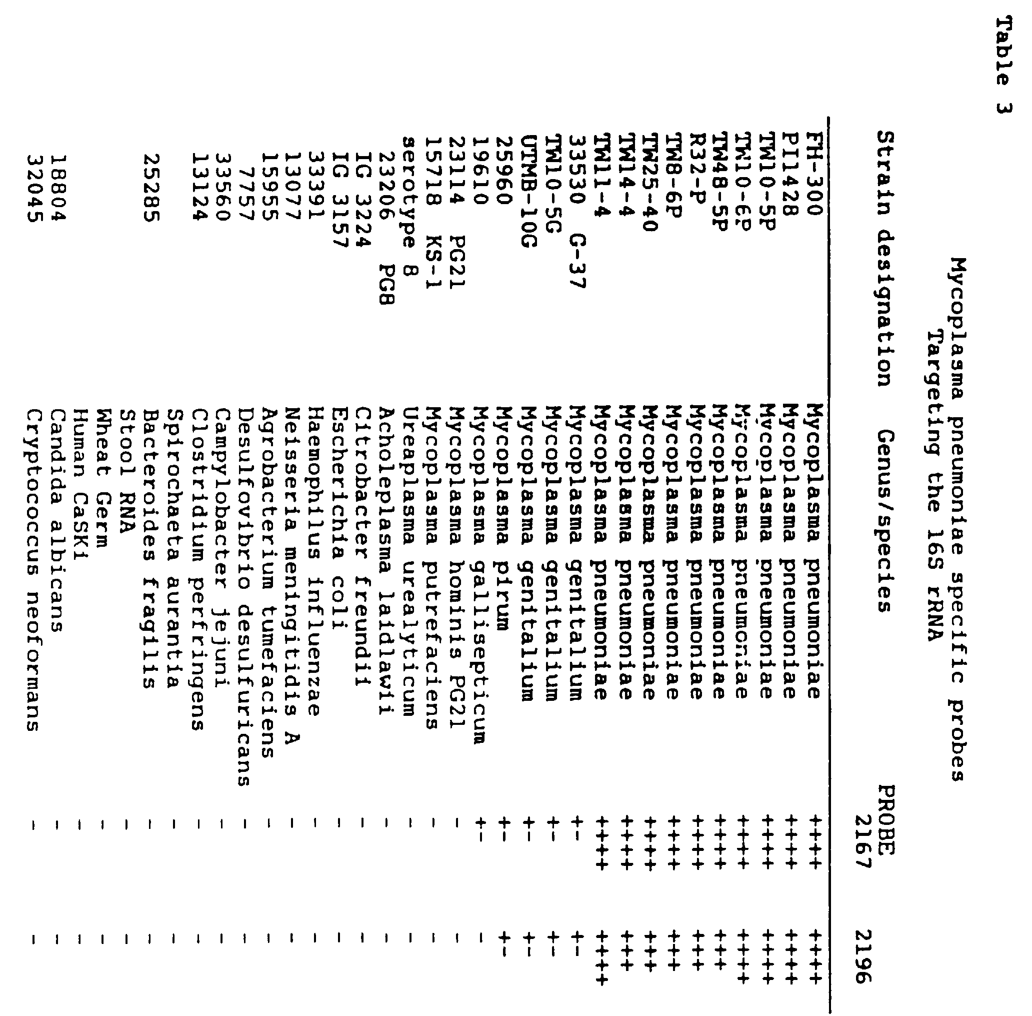

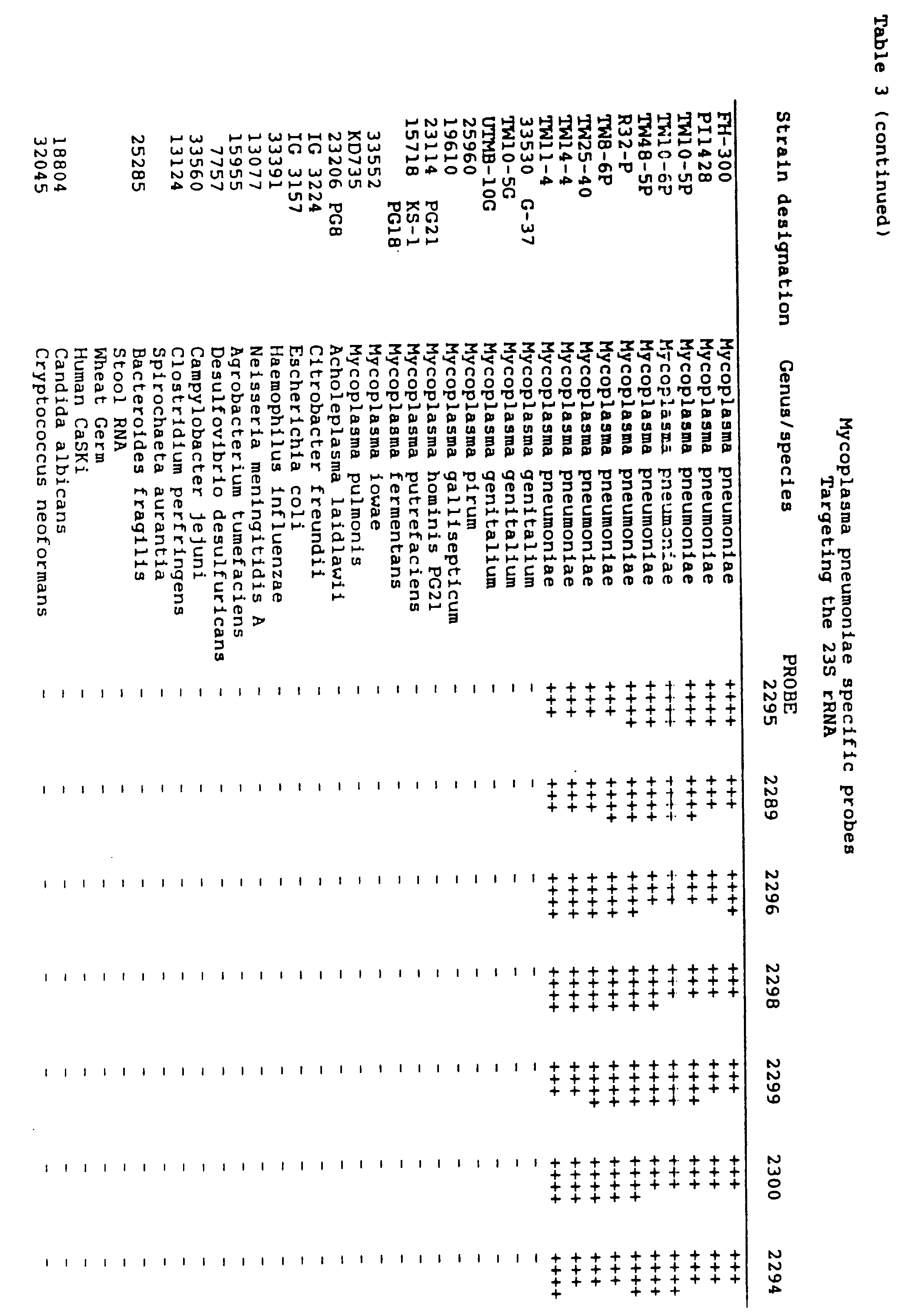

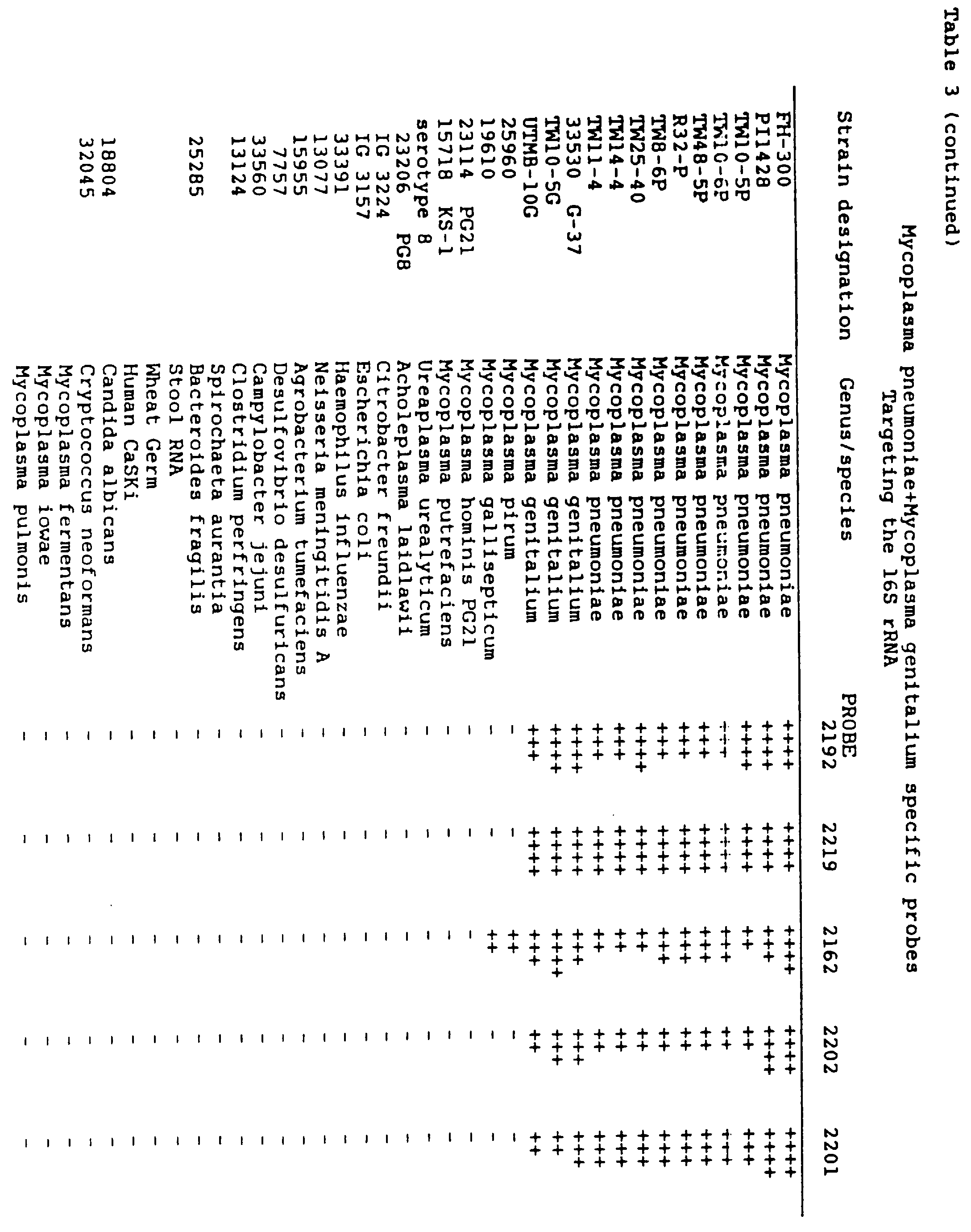

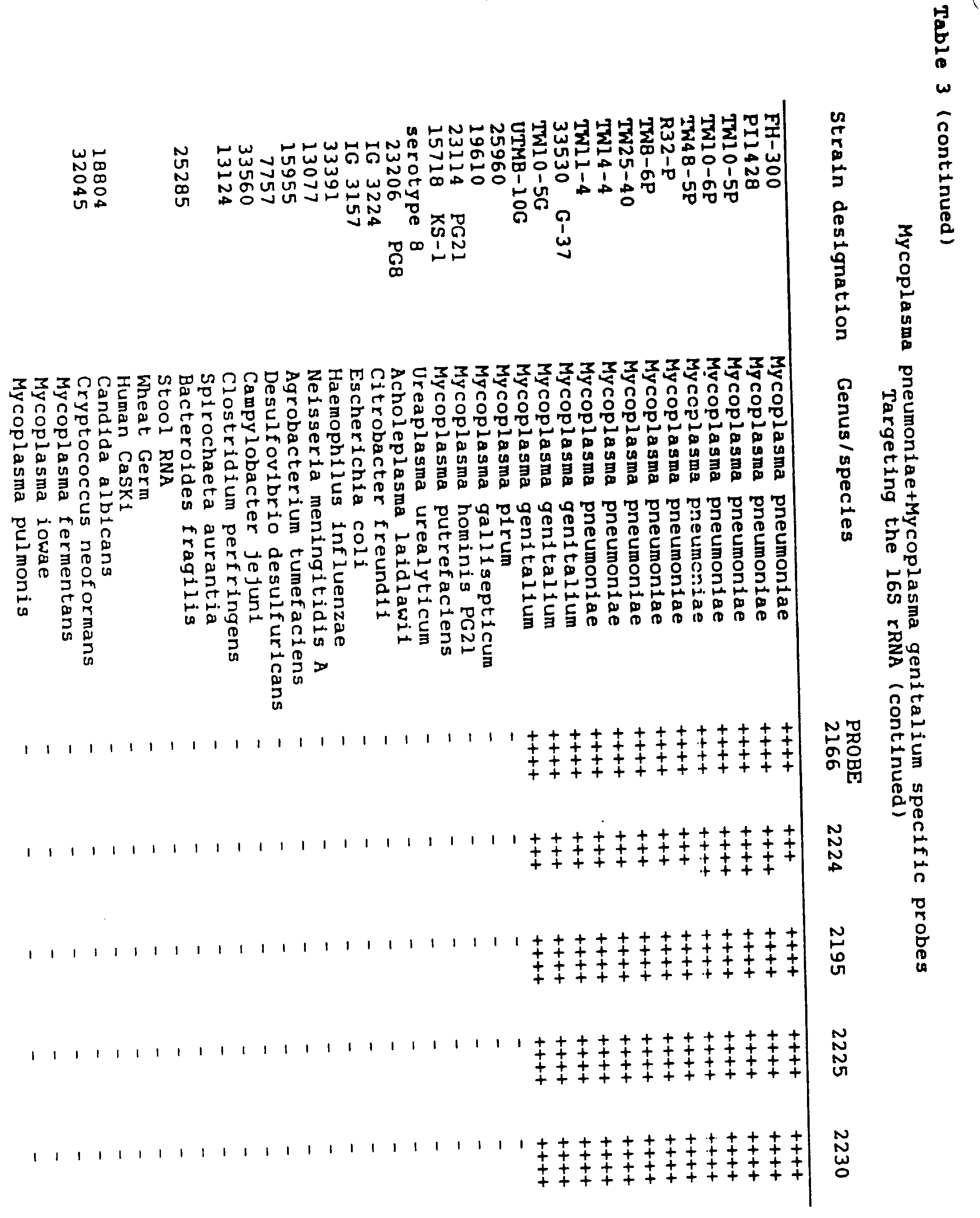

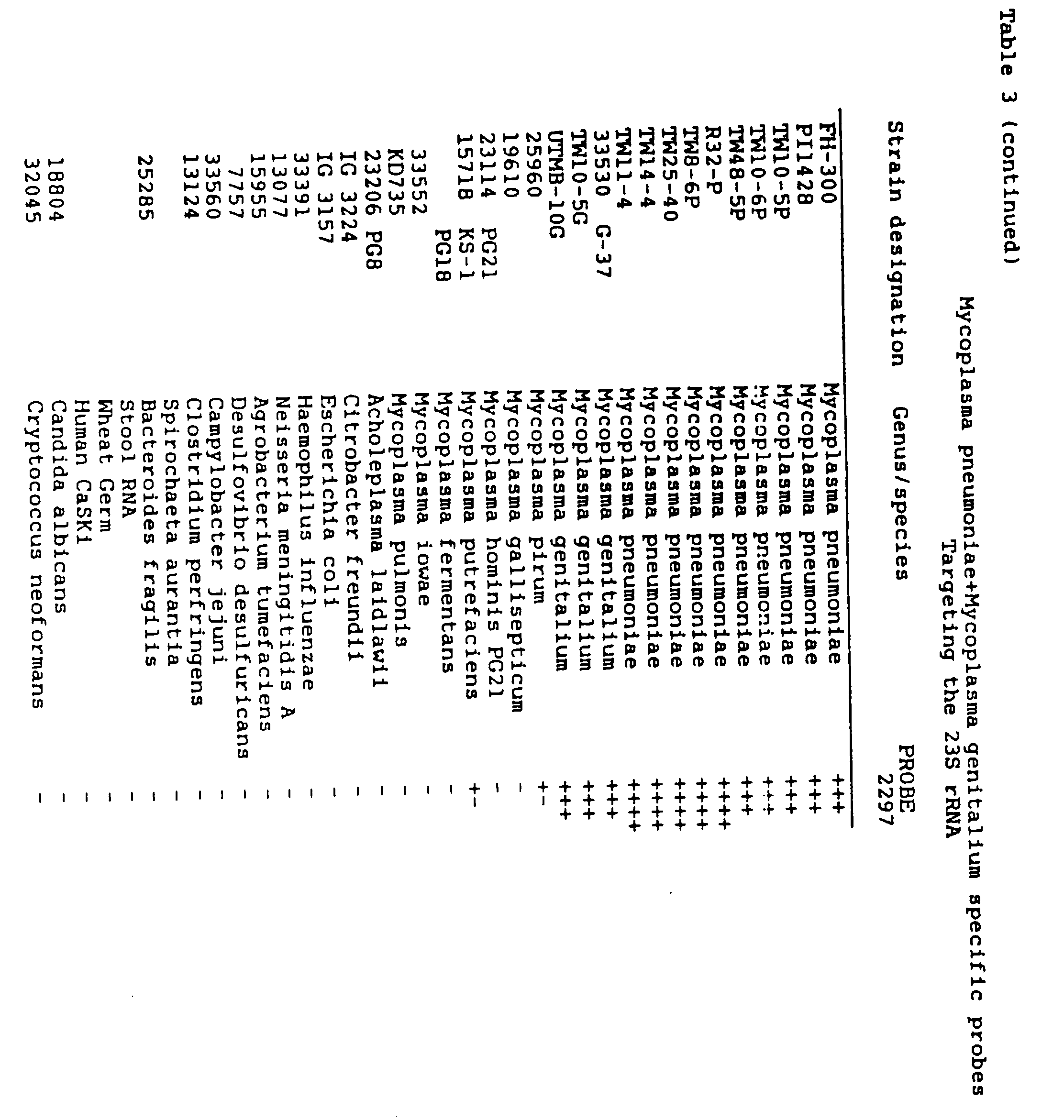

The 10 representative strains of Mycoplasma pneumoniae and 3 strains of M. genitalium represent a selection from the National Institute of Allergy and Infectious Disease's Mycoplasma Section. Additional species of bacteria and a few fungi were added to the panel in order to represent the breadth of known bacterial taxa. Mycoplasma gallisepticum is critically important as a representative close relative of the M. pneumoniae/M. genitalium cluster.

All species on the panel are represented by 100 ng of purified, denatured RNA. Probes were synthesized (by standard methods and chemistries well known to those skilled in the art), ³²Phosphorous labelled, hybridized to panels under standard conditions, at the temperatures indicated, and autoradiographically evaluated. "++++" represents strongest hybridization signal after three hours exposure, with slightly lighter signal represented by "+++", diminishing to "++", then "+". "+-" is virtually absent, and "-" is indicative of no hybridization of probe to target. The specificity is empirical and in the case of several of the M. pneumoniae + M. genitalium probes represents a slight departure from the predicted specific behavior.

### Example 2. Dual Probe Hybridization

In actual practice, many applications of these probes would employ a pair of probes being used simultaneously in a "sandwich" hybridization scheme. Turning now to Figure 1, a sandwich assay is depicted featuring a capture probe 12 and a detector probe 13. The capture probe 12 ideally would be a bifunctional nucleic acid manufactured by adding a homopolymeric 3' tail to a probe with high target specificity. The tail would, in turn, hybridize to the complementary homopolymer 11 on a solid surface 10, such as a glass bead or a filter disc. Hybridization of the capture probe 12 to its target 15, in this case mycoplasma rRNA, would complex the target 15 with the solid support 10. The detector probe 13, advantageously also with some degree of specificity, would be part of a detection scheme relying on radioactivity, fluorescence. chemiluminescence, color, and other detection means, and including detection moiety 14 which would report the presence of the entire hybridization complex. The detector probe could potentially be incorporated as an RNA sequence into an amplifiable Q-beta midivariant as described by Kramer and Lizardi (Nature, vol. 339, 1989).

### Example 3. Clinical diagnosis of Mycoplasma pneumoniae primary atypical pneumonia

A clinical sample, such as a swab, sputum, or tissue is processed so as to liberate the total nucleic acid content. The sample, containing disrupted mycoplasmas is incubated in the presence of capture probe, detector probe, and magnetic particle beads which have been derivatized with oligo-Thymidine--as in Example 2--in a chaotropic buffer such as guanidinium isothiocyanate.

If target molecules, Mycoplasma pneumoniae or M. genitalium 16S or 23S rRNA (depending on which probe sets are employed) are present, a Bead + Capture Probe + Target + Detector Probe hybridization complex is formed, as in Figure 1. The presence of a magnet near the bottom of the reaction tube will cause the magnetic particle + hybridization complex to adhere to the side of the tube enabling removal of the sample matrix, unbound probe, etc. Repeated rehydration and denaturation of the bead+probe+ target complex would enable significant background reduction (as described in USSN 922,155, Collins, 1986). In this example, final detection could entail spotting the beads on a membrane and assaying by autoradiography. Alternatively, the detector probe could be an amplifiable midivariant probe.

For this particular assay, the following capture and detector probes are examples of preferred pairs:
Probe 2196 + Probe 2167, Probe 2162 + Probe 2202
Probe 2224 + Probe 2166, Probe 2162 + Probe 2230
Probe 2167 + Probe 2219, Probe 2192 + Probe 2202
Probe 2296 + Probe 2297, Probe 2299 + Probe 2300

### Example 4. Clinical diagnosis of M. pneumoniae primary atypical pneumonia from human sample employing polymerase chain reaction amplification of mycoplasma rDNA

Sample processing is designed so as to yield DNA. One of the probes described herein is used in conjunction with the antiparallel complement of one of the probes described herein to enzymatically amplify a segment of Mycoplasma pneumoniae or M. genitalium gene encoding mycoplasma rRNA in a polymerase chain reaction. Resultant material can then be assayed in a "sandwich" hybridization assay with any of the probes described herein. The polymerase chain reaction can, itself, be made either highly specific by employing probe/primers described herein, or the reaction can be made more general using less specific probes, and identifying the amplification product as M. pneumoniae with probes described herein which have great specificity.

### Example 5. In situ hybridization as a cytological stain

The probes of the present invention can be employed as cytological staining reagents. For example, a sputum sample is applied to a microscope slide. After appropriate fixation and lysis, hybridization of probes is carried out in situ. In this example, Mycoplasma pneumoniae could be visualized in a specimen by fluorescently labelling Probe 2167 and examining the slide using a fluorescent microscope, looking for small fluorescent bodies.

### Example 6. Confirmation of Mycoplasma pneumoniae following culture

Following a standard cultivation step for Mycoplasma pneumoniae or M. genitalium, for example on H-agar plates or SP-6 broth, a colony or liquid culture is tested for the presence of M. pneumoniae by employing Probes 2167 and 2196.

## Claims

1. A nucleic acid probe that hybridizes stably to the 16S rRNA or DNA encoding 16S rRNA of *Mycoplasma pneumoniae* and does not hybridize stably to the 16S rRNA or DNA encoding 16S rRNA of non-*Mycoplasma* bacteria and humans under identical hybridization conditions, the probe having a sequence complementary to or homologous with at least a portion of the sequence of Probe 2167 (CCAATTTGCATTAGCAGTCTCGCTAGACAATGTAACT).

2. A nucleic acid probe of claim 1 having a sequence complementary to or homologous with the sequence of Probe 2167.

3. A nucleic acid probe that hybridizes stably to the 23S rRNA or DNA encoding 23S rRNA of *Mycoplasma pneumoniae* and does not hybridize stably to the 23S rRNA or DNA encoding 23S rRNA of non-*Mycoplasma* bacteria and humans under identical hybridization conditions, the probe having a sequence complementary to or homologous with at least a portion of the sequence of any one of Probe 2295 (TCACCACGTATTGCTTTAATTGACTATTTATTCATCAAT), Probe 2289 (CAACCCCTATCTAATGATAAGTTTGGCCTGT), Probe 2296 (TTCTATCGTTTTCAAGTC CACATTGCAAGCCCTAC), Probe 2298 (TGGTTACAGCTAGATTAACCCTAGAAGCTTTT CT), Probe 2299 (CCTATTCTCTACATGATAATGTCCTGATCAATATT), Probe 2300 (TAGTATTCCACCTTTCGCATCAACAAGTCCTAGCGAACT), or Probe 2294 (TAGAAGC AACACTCTTCAATCTTCCTACGGGCACAAT).

4. A nucleic acid probe of claim 3 having a sequence complementary to or homologous with the sequence of any one of Probe 2295, Probe 2289, Probe 2296, Probe 2298, Probe 2299, Probe 2300, or Probe 2294.

5. A nucleic acid probe that hybridizes stably to the 16S rRNA or DNA encoding 16S rRNA of *Mycoplasma pneumoniae* and *Mycoplasma genitalium* and does not hybridize stably to the 16S rRNA or DNA encoding 16S rRNA of non-Mycoplasma bacteria and humans under identical hybridization conditions, the probe having a sequence complementary to or homologous with at least a portion of the sequence of Probe 2202 (CTTTGATTCATGCGAACCAAAGTTCTTATGCGGTATTAGC TAGTCTTTC), Probe 2166 (CTCCCTACCACACTCTAGATTAATAGTTTCCAATGC), Probe 2225 (CTTTTCAGATTTGCTCACTTTTACAAGTTGGCTACTGT), or Probe 2230 (CTTTAATTCATGCGAACTAAAGTTCTTATGCGGTATTAGCTAGTTTTC).

6. A nucleic acid probe of claim 5 having a sequence complementary to or homologous with the sequence of any one of Probe 2202, Probe 2166, Probe 2225, or Probe 2230.

7. A nucleic acid probe that hybridizes stably to the 23S rRNA or DNA encoding 23S rRNA of *Mycoplasma pneumoniae* and *Mycoplasma genitalium* and does not hybridize stably to the 23S rRNA or DNA encoding 23S rRNA of non-Mycoplasma bacteria and humans under identical hybridization conditions, the probe having a sequence complementary to or homologous with at least a portion of the sequence of Probe 2297 (GCTATCACCCTTTTGCGCGCTGCTTTCCAAT).

8. A nucleic acid probe of claim 7 having a sequence complementary to or homologous with the sequence of Probe 2297.

9. A set of nucleic acid probes comprising at least two nucleic acid probes, each probe hybridizing stably to the 16S rRNA or DNA encoding 16S rRNA of *Mycoplasma pneumoniae* and not hybridizing stably to the 16S rRNA or DNA encoding 16S rRNA of non-Mycoplasma bacteria and humans under identical hybridization conditions, ana each probe having a different base sequence composition, wherein a first nucleic acid probe has a sequence complementary to or homologous with at least a portion of the sequence of any one of Probe 2167 (CCAATTTGCATTAGCAGTCTCGCTAGACAATGTAACT), Probe 2202 Probe 2166 (CTCCCTACCACACTCTAGATTAATAGTTTCCAATGC), Probe 2202 (CTTTGATTCATGCGAACCAAAGTTCTTATGCGGTATTAGCTAGTCTTTC) or Probe 2230 (CTTTAATTCATGCGAACTAAAGTTCTTATGCGGTATTAGCTAGTTTTC), and a second nucleic acid probe has a sequence complementary to or homologous with at least a portion of the sequence of any one of Probe 2167 Probe 2162 (CGCCTCTAAAGTATTACTACTTTCGATCGACTTGCATGT), Probe 2166, Probe 2196 (CTTTACAGATTTGCTCACTTTTACAAGCTGGCGACTGT), Probe 2192 (CCCTCATCAAATAACGAACCCTTGCAGGTCCT), Probe 2219 (CCTCCATTATGTTTCCATAACTTTGCCAAGGATGT), Probe 2201 (CAAAATGGTACAGTC AAACTCTAGCCATTACCTGCTAAAGTCATTC), Probe 2224 (CACTACCGAGGGGATCGCC CCGACAGCTAGTATCTATCGT), Probe 2195 (GCTCACTTTTACAAGCTGGCGACTGTTTG TATTGGCC) or Probe 2225 (CTTTTCAGATTTGCTCACTTTTACAAGTTGGCTACTGT).

10. A set of nucleic acid probes of claim 9 selected from the group of probe sets
Probe 2196 and Probe 2167, Probe 2162 and Probe 2202,
Probe 2224 and Probe 2166, Probe 2162 and Probe 2230,
Probe 2167 and Probe 2219, or Probe 2192 and Probe 2202.

11. A set of nucleic acid probes comprising at least two nucleic acid probes, each probe hybridizing stably to the 23S rRNA or DNA encoding 23S rRNA of *Mycoplasma pneumoniae* and not hybridizing stably to the 23S rRNA or DNA encoding 23S rRNA of non-Mycoplasma bacteria and humans under identical hybridization conditions, and each probe having a different base sequence composition and having a sequence complementary to or homologous with at least a portion of the sequence of any one of Probe 2295, Probe 2289, Probe 2296, Probe 2298, Probe 2299, Probe 2300, Probe 2294, or Probe 2297.

12. A set of nucleic acid probes of claim 11 selected from the group of probe sets
Probe 2296 and Probe 2297, or Probe 2299 and Probe 2300.

13. A method for detecting the presence of either one or both of *Mycoplasma pneumoniae* and *Mycoplasma genitalium* in a sample comprising:
a) contacting said sample with at least one nucleic acid probe of any one of claims 1, 3, 5, or 7;
b) imposing hybridization conditions on the sample and said nucleic acid probe to allow said probe to hybridize stably to the rRNA or DNA encoding rRNA of either one or both of Mycoplasma *pneumoniae* and *Mycoplasma genitalium,* if present in the sample, to form nucleic acid complexes, under conditions which do not allow said probe to form stable hybridized nucleic acid complexes with non-*Mycoplasma* bacteria; and
c) detecting said nucleic acid complexes as an indication of the presence of either one or both of *Mycoplasma pneumoniae* and *Mycoplasma genitalium* in the sample.

14. A method of claim 13 wherein said nucleic acid probe of said contacting step is complementary to or homologous with a sequence of any one of Probe 2167, Probe 2295, Probe 2289, Probe 2296, Probe 2298, Probe 2299, Probe 2300, Probe 2294, Probe 2202, Probe 2166, Probe 2225, Probe 2230, or Probe 2297.

15. A method for detecting the presence of either one or both of *Mycoplasma pneumoniae* and *Mycoplasma genitalium* in a sample comprising:
a) contacting said sample with a set of at least two nucleic acid probes of any one of claims 9 or 11;
b) imposing hybridization conditions on the sample and said nucleic acid probes to allow said probes to hybridize stably to the rRNA or DNA encoding rRNA of either one or both of Mycoplasma *pneumoniae* and *Mycoplasma genitalium,* if present in the sample, to form nucleic acid complexes, under conditions which do not allow said probe to form stable hybridized nucleic acid complexes with non-*Mycoplasma* bacteria; and
c) detecting said nucleic acid complexes as an indication of the presence of either one or both of *Mycoplasma pneumoniae* and *Mycoplasma genitalium* in the sample.

16. A method of claim 15 wherein said set of nucleic acid probes is selected from the group of probe sets
Probe 2196 and Probe 2167, Probe 2162 and Probe 2202,
Probe 2224 and Probe 2166, Probe 2162 and Probe 2230,
Probe 2167 and Probe 2219, Probe 2192 and Probe 2202,
Probe 2296 and Probe 2297, or Probe 2299 and Probe 2300.

17. A kit for detecting either one or both of Mycoplasma pneumoniae and Mycoplasma genitalium associated with human primary atypical pneumonia, comprising a nucleic acid probe as defined in claims 1 to 8 or a set of nucleic acid probes as defined in claim 9 to 12.

## Patentansprüche

1. Nucleinsäuresonde, die stabil mit der 16S rRNA oder der für die 16S rRNA codierenden DNA von Mycoplasma pneumoniae hybridisiert und unter identischen Hybridisierungsbedingungen mit der 16S rRNA oder der für 16S rRNA codierenden DNA von Nicht-Mycoplasmabakterien und von Menschen nicht stabil hybridisiert, wobei die Sonde eine Sequenz besitzt, die komplementär oder homolog zu wenigstens einer Teilsequenz der Sonde 2167 (CCAATTTGCATTAGCAGTCTCGCTAGACAATGTAACT) ist.

2. Nucleinsäuresonde nach Anspruch 1 mit einer Sequenz, die komplementär oder homolog zu der Sequenz der Sonde 2167 ist.

3. Nucleinsäuresonde, die stabil mit der 23S rRNA oder der für 23S rRNA codierenden DNA von Mycoplasma pneumoniae hybridisiert und unter identischen Hybridisierungsbedingungen mit der 23S rRNA oder der für 23S rRNA codierenden DNA von Nicht-Mycoplasmabakterien und von Menschen nicht stabil hybridisiert, wobei die Sonde eine Sequenz besitzt, die komplementär oder homolog zu wenigstens einer Teilsequenz der Sonde 2295 (TCACCACGTATTGCTTTAATTGACTATTTATTCATCAAT), der Sonde 2289 (CAACCCCTATCTAATGATAAGTTTGGCCTGT), der Sonde 2296 (TTCTATCGTT TTCAAGTCCACATTGCAAGCCCTAC), der Sonde 2298 (TGGTTACAGCTAGAT TAACCCTAGAAGCTTTTCT), der Sonde 2299 (CCTATTCTCTACATGA TAATGTCCTGATCAATATT), der Sonde 2300 (TAGTATTCCACCTTTCGCATCAA CAAGTCCTAGCGAACT) oder der Sonde 2294 (TAGAAGCAACACTCTT CAATCTTCCTACGGGCACAAT) ist.

4. Nucleinsäuresonde nach Anspruch 3 mit einer Sequenz, die komplementär oder homolog zu der Sequenz der Sonde 2295, der Sonde 2289, der Sonde 2296, der Sonde 2298, der Sonde 2299, der Sonde 2300 oder der Sonde 2294 ist.

5. Nucleinsäuresonde, die stabil mit der 16S rRNA oder der für die 16S rRNA codierenden DNA von Mycoplasma pneumoniae und von Mycoplasma genitalium hybridisiert und unter identischen Hybridisierungsbedingungen mit der 16S rRNA oder der für die 16S rRNA codierenden DNA von Nicht-Mycoplasmabakterien und von Menschen nicht stabil hybridisiert, wobei die Sonde eine Sequenz besitzt, die komplementär oder homolog zu wenigstens einer Teilsequenz der Sonde 2202 (CTTTGATTCATGCGAACCAAAGTTCT TATGCGGTATTAGCTAGTCTTTC), der Sonde 2166 (CTCCCTACCACACTCTA GATTAATAGTTTCCAATGC), der Sonde 2225 (CTTTTCAGATTTGCTCACTTT TACAAGTTGGCTACTGT) oder der Sonde 2230 (CTTTAATTCATGCGAACTAA AGTTCTTATGCGGTATTAGCTAGTTTTC) ist.

6. Nucleinsäuresonde nach Anspruch 5 mit einer Sequenz, die komplementär oder homolog zu der Sequenz der Sonde 2202, der Sonde 2166, der Sonde 2225 oder der Sonde 2230 ist.

7. Nucleinsäuresonde, die stabil mit der 23S rRNA oder der für die 23S rRNA codierenden DNA von Mycoplasma pneumoniae und von Mycoplasma genitalium hybridisiert und unter identischen Hybridisierungsbedingungen mit der 23S rRNA oder der für die 23S rRNA codierenden DNA von Nicht-Mycoplasmabakterien und von Menschen nicht stabil hybridisiert, wobei die Sonde eine Sequenz besitzt, die komplementär oder homolog zu wenigstens einer Teilsequenz der Sonde 2297 (GCTATCACCCTTTTGCGCGCTGCTTT CCAAT) ist.

8. Nucleinsäuresonde nach Anspruch 7 mit einer Sequenz, die komplementär oder homolog zu der Sequenz der Sonde 2297 ist.

9. Nucleinsäuresondenset, das wenigstens zwei Nucleinsäuresonden umfaßt, wobei jede Sonde stabil mit der 16S rRNA oder der für die 16S rRNA codierenden DNA von Mycoplasma pneumoniae hybridisiert und unter identischen Hybridisierungsbedingungen mit der 16S rRNA oder der für die 16S rRNA codierenden DNA von Nicht-Mycoplasmabakterien und von Menschen nicht stabil hybridisiert, und wobei jede Sonde eine andere Basensequenz besitzt, worin eine erste Nucleinsäuresonde eine Sequenz besitzt, die komplementär oder homolog zu wenigstens einer Teilsequenz der Sonde 2167 (CCAATTTGCATTAGCAGTCTCGCTAGA CAATGTAACT), der Sonde 2166 (CTCCCTACCACACTCTAGATTAA TAGTTTCCAATGC), der Sonde 2202 (CTTTGATTCATGCGAACCAAAGTTCT TATGCGGTATTAGCTAGTCTTTC) oder der Sonde 2230 (CTTTAATT CATGCGAACTAAAGTTCTTATGCGGTATTAGCTAGTTTTC) ist, und eine zweite Nucleinsäuresonde eine Sequenz besitzt, die komplementär oder homolog zu wenigstens einer Teilsequenz der Sonde 2167, der Sonde 2162 (CGCCTCTAAAGTATTACTACTTTCGATCGACTTGCATGT), der Sonde 2166, der Sonde 2196 (CTTTACAGATTTGCTCACTTTTACAAGCTGGCG ACTGT), der Sonde 2192 (CCCTCATCAAATAACGAACCCTTGCAGGTCCT), der Sonde 2219 (CCTCCATTATGTTTCCATAACTTTGCCAAGGATGT), der Sonde 2201 (CAAAATGGTACAGTCAAACTCTAGCCATTACCTGCTAAAGTCATTC), der Sonde 2224 (CACTACCGAGGGGATCGCCCCGACAGCTAGTATCTATCGT), der Sonde 2195 (GCTCACTTTTACAAGCTGGCGACTGTTTGTATTGGCC) oder der Sonde 2225 (CTTTTCAGATTTGCTCACTTTTACAAGTTGGCTACTGT) ist.

10. Nucleinsäuresondenset nach Anspruch 9, ausgewählt aus der Gruppe der Sondensets:
Sonde 2196 und Sonde 2167, Sonde 2162 und Sonde 2202,
Sonde 2224 und Sonde 2166, Sonde 2162 und Sonde 2230,
Sonde 2167 und Sonde 2219, oder Sonde 2192 und Sonde 2202.

11. Nucleinsäuresondenset, das wenigstens zwei Nucleinsäuresonden umfaßt, wobei jede Sonde stabil mit der 23S rRNA oder der für die 23S rRNA codierenden DNA von Mycoplasma pneumoniae hybridisiert und unter identischen Hybridisierungsbedingungen mit der 23S rRNA oder der für die 23S rRNA codierenden DNA von Nicht-Mycoplasmabakterien und von Menschen nicht stabil hybridisiert, und wobei jede Sonde eine andere Basensequenz besitzt und zwar eine Sequenz, die komplementär oder homolog zu wenigstens einer Teilsequenz der Sonde 2295, der Sonde 2289, der Sonde 2296, der Sonde 2298, der Sonde 2299, der Sonde 2300, der Sonde 2294 oder der Sonde 2297 ist.

12. Nucleinsäuresondenset nach Anspruch 11, ausgewählt aus der Gruppe von Sondensets:
Sonde 2296 und Sonde 2297, oder Sonde 2299 und Sonde 2300.

13. Verfahren zum Nachweis von Mycoplasma pneumoniae und/oder Mycoplasma genitalium in einer Probe, das umfaßt:
(a) Inkontaktbringen der Probe mit wenigstens einer Nucleinsäuresonde nach irgendeinem der Ansprüche 1, 3, 5 oder 7;
(b) Einstellen von Hybridisierungsbedingungen in der Probe für die Nucleinsäuresonde, damit die Sonde mit der rRNA oder der für die rRNA codierenden DNA von Mycoplasma pneumoniae und/oder Mycoplasma genitalium, falls in der Probe vorhanden, unter Bedingungen, unter denen die Sonde keine stabilen Nucleinsäurehybridkomplexe mit Nicht-Mycoplasmabakterien bildet, stabil zu Nucleinsäurekomplexen hybridisieren kann; und
(c) Nachweis der Nucleinsäurekomplexe als Zeichen für das Vorhandensein von Mycoplasma pneumoniae und/oder Mycoplasma genitalium in der Probe.

14. Verfahren nach Anspruch 13, worin die Nucleinsäuresonde in der Kontaktstufe komplementär oder homolog zu der Sequenz der Sonde 2167, der Sonde 2295, der Sonde 2289, der Sonde 2296, der Sonde 2298, der Sonde 2299, der Sonde 2300, der Sonde 2294, der Sonde 2202, der Sonde 2166, der Sonde 2225, der Sonde 2230 oder der Sonde 2297 ist.

15. Verfahren zum Nachweis von Mycoplasma pneumoniae und/oder Mycoplasma genitalium in einer Probe, das umfaßt:
(a) Inkontaktbringen der Probe mit einem Set aus wenigstens zwei Nucleinsäuresonden nach irgendeinem der Ansprüche 9 oder 11;
(b) Einstellen von Hybridisierungsbedingungen in der Probe für die Nucleinsäuresonden, damit die Sonden mit der rRNA oder der für die rRNA codierenden DNA von Mycoplasma pneumoniae und/oder Mycoplasma genitalium, falls in der Probe vorhanden, unter Bedingungen, unter denen die Sonden keine stabilen Nucleinsäurehybridkomplexe mit Nicht-Mycoplasmabakterien bilden, stabil zu Nucleinsäurekomplexen hybridisieren können; und
(c) Nachweis der Nucleinsäurekomplexe als Zeichen für das Vorhandensein von Mycoplasma pneumoniae und/oder Mycoplasma genitalium in der Probe.

16. Verfahren nach Anspruch 15, worin das Nucleinsäuresondenset ausgewählt ist aus der Gruppe von Sondensets:
Sonde 2196 und Sonde 2167, Sonde 2162 und Sonde 2202,
Sonde 2224 und Sonde 2166, Sonde 2162 und Sonde 2230,
Sonde 2167 und Sonde 2219, Sonde 2192 und Sonde 2202,
Sonde 2296 und Sonde 2297, oder Sonde 2299 und Sonde 2300.

17. Kit zum Nachweis von Mycoplasma pneumoniae und/oder Mycoplasma genitalium bei atypischer Pneumonie beim Menschen, der eine Nucleinsäuresonde nach den Ansprüchen 1 bis 8 oder ein Nucleinsäuresondenset nach den Ansprüchen 9 bis 12 umfaßt.

## Revendications

1. Sonde d'acides nucléiques qui s'hybride de façon stable à l'ARNr 16S ou à l'ADN codant pour l'ARNr 16S de *Mycoplasma pneumoniae* et qui ne s'hybride pas de façon stable à l'ARNr 16S ni à l'ADN codant pour l'ARNr 16S de bactéries non *Mycoplasma* et humain dans des conditions d'hybridation identiques, la sonde ayant une séquence complémentaire ou homologue à au moins une partie de la séquence de la Sonde 2167 (CCAATTTGCATTAGCAGTCTCGCTAGACAATGTAACT).

2. Sonde d'acides nucléiques selon la revendication 1 ayant une séquence complémentaire ou homologue à la séquence de la Sonde 2167.

3. Sonde d'acides nucléiques qui s'hybride de façon stable à l'ARNr 23S ou à l'ADN codant pour l'ARNr 23S de *Mycoplasma pneumoniae* et qui ne s'hybride pas de façon stable à l'ARNr 23S ni à l'ADN codant pour l'ARNr 23S de bactéries non *Mycoplasma* et humain dans des conditions d'hybridation identiques, la sonde ayant une séquence complémentaire ou homologue à au moins une partie de la séquence d'une sonde quelconque parmi la Sonde 2295 (TCACCACGTATTGCTTTAATTGACTATTTATTCATCAAT), la Sonde 2289 (CAACCCCTATCTAATGATAAGTTTGGCCTGT), la Sonde 2296 (TTCTATCGTTTTCAAGTCCACATTGCAAGCCCTAC), la Sonde 2298 (TGGTTACAGCTAGATTAACCCTAGAAGCTTTTCT), la Sonde 2299 (CCTATTCTCTACATGATAATGTCCTGATCAATATT), la Sonde 2300 (TAGTATTCCACCTTTCGCATCAACAAGTCCTAGCGAACT), ou la Sonde 2294 (TAGAAGCAACACTCTTCAATCTTCCTACGGGCACAAT).

4. Sonde d'acides nucléiques selon la revendication 3 ayant une séquence complémentaire ou homologue à la séquence d'une sonde quelconque parmi la Sonde 2295, la Sonde 2289, la Sonde 2296, la Sonde 2298, la Sonde 2299, la Sonde 2300, ou la Sonde 2294.

5. Sonde d'acides nucléiques qui s'hybride de façon stable à l'ARNr 16S ou à l'ADN codant pour l'ARNr 16S de *Mycoplasma pneumoniae* et de *Mycoplasma genitalium* et qui ne s'hybride pas de façon stable à l'ARNr 16S ni à l'ADN codant pour l'ARNr 16S de bactéries non *Mycoplasma* et humain dans des conditions d'hybridation identiques, la sonde ayant une séquence complémentaire ou homologue à au moins une partie de la séquence de la Sonde 2202 (CTTTGATTCATGCGAACCAAAGTTCTTATGCGGTATTAGCTAGTCTTTC), de la Sonde 2166 (CTCCCTACCACACTCTAGATTAATAGTTTCCAATGC), de la Sonde 2225 (CTTTTCAGATTTGCTCACTTTTACAAGTTGGCTACTGT), ou de la Sonde 2230 (CTTTAATTCATGCGAACTAAAGTTCTTATGCGGTATTAGCTAGTTTTC).

6. Sonde d'acides nucléiques selon la revendication 5 ayant une séquence complémentaire ou homologue à la séquence d'une sonde quelconque parmi la Sonde 2202, la Sonde 2166, la Sonde 2225 ou la Sonde 2230.

7. Sonde d'acides nucléiques qui s'hybride de façon stable à l'ARNr 23S ou à l'ADN codant pour l'ARNr 23S de *Mycoplasma pneumoniae* et de *Mycoplasma genitalium* et qui ne s'hybride pas de façon stable à l'ARNr 23S ni à l'ADN codant pour l'ARNr 23S de bactéries non *Mycoplasma* et humain dans des conditions d'hybridation identiques, la sonde ayant une séquence complémentaire ou homologue à au moins une partie de la séquence de la Sonde 2297 (GCTATCACCCTTTTGCGCGCTGCTTTCCAAT).

8. Sonde d'acides nucléiques selon la revendication 7 ayant une séquence complémentaire ou homologue à la séquence de la Sonde 2297.

9. Ensemble de sondes d'acides nucléiques comprenant au moins deux sondes d'acides nucléiques, chaque sonde s'hybridant de façon stable à l'ARNr 16S ou à l'ADN codant pour l'ARNr 16S de *Mycoplasma pneumoniae* et ne s'hybridant pas de façon stable à l'ARNr 16S ni à l'ADN codant pour l'ARNr 16S de bactéries non Mycoplasma et humain dans des conditions d'hybridation identiques, et chaque sonde ayant une composition de séquence de bases différente, dans lequel une première sonde d'acides nucléiques possède une séquence complémentaire ou homologue à au moins une partie de la séquence d'une sonde quelconque parmi la Sonde 2167 (CCAATTTGCATTAGCAGTCTCGCTAGACAATGTAACT), la Sonde 2166 (CTCCCTACCACACTCTAGATTAATAGTTTCCAATGC), la Sonde 2202 (CTTTGATTCATGCGAACCAAAGTTCTTATGCGGTATTAGCTAGTCTTTC) ou la Sonde 2230 (CTTTAATTCATGCGAACTAAAGTTCTTATGCGGTATTAGCTAGTTTTC), et une deuxième sonde d'acides nucléiques possède une séquence complémentaire ou homologue à au moins une partie de la séquence d'une sonde quelconque parmi la Sonde 2167, la Sonde 2162 (CGCCTCTAAAGTATTACTACTTTCGATCGACTTGCATGT), la Sonde 2166, la Sonde 2196 (CTTTACAGATTTGCTCACTTTTACAAGCTGGCGACTGT), la Sonde 2192 (CCCTCATCAAATAACGAACCCTTGCAGGTCCT), la Sonde 2219 (CCTCCATTATGTTTCCATAACTTTGCCAAGGATGT), la Sonde 2201 (CAAAATGGTACAGTCAAACTCTAGCCATTACCTGCTAAAGTCATTC), la Sonde 2224 (CACTACCGAGGGGATCGCCCCGACAGCTAGTATCTATCGT), la Sonde 2195 (GCTCACTTTTACAAGCTGGCGACTGTTTGTATTGGCC) ou la Sonde 2225 (CTTTTCAGATTTGCTCACTTTTACAAGTTGGCTACTGT).

10. Ensemble de sondes d'acides nucléiques selon la revendication 9 choisi dans le groupe constitué par les ensembles de sondes
Sonde 2196 et Sonde 2167, Sonde 2162 et Sonde 2202, Sonde 2224 et Sonde 2166, Sonde 2162 et Sonde 2230, Sonde 2167 et Sonde 2219, ou Sonde 2192 et Sonde 2202.

11. Ensemble de sondes d'acides nucléiques comprenant au moins deux sondes d'acides nucléiques, chaque sonde s'hybridant de façon stable à l'ARNr 23S ou à l'ADN codant pour l'ARNr 23S de *Mycoplasma pneumoniae* et ne s'hybridant pas de façon stable à l'ARNr 23S ni à l'ADN codant pour l'ARNr 23S de bactéries non *Mycoplasma* et humain dans des conditions d'hybridation identiques, et chaque sonde ayant une composition de séquence de bases différente et une séquence complémentaire ou homologue à au moins une partie de la séquence d'une sonde quelconque parmi la Sonde 2295, la Sonde 2289, la Sonde 2296, la Sonde 2298, la Sonde 2299, la Sonde 2300, la Sonde 2294, ou la Sonde 2297.

12. Ensemble de sondes d'acides nucléiques selon la revendication Il choisi dans le groupe constitué par les ensembles de sondes
Sonde 2296 et Sonde 2297, ou Sonde 2299 et Sonde 2300.

13. Procédé pour détecter la présence soit de *Mycoplasma pneumoniae,* soit de *Mycoplasma genitalium,* soit des deux à la fois dans un échantillon, comprenant les étapes qui consistent à:
a) mettre ledit échantillon au contact d'au moins une sonde d'acides nucléiques selon l'une quelconque des revendications 1, 3, 5 ou 7;
b) imposer des conditions d'hybridation sur l'échantillon et ladite sonde d'acides nucléiques pour permettre à ladite sonde de s'hybrider de façon stable à l'ARNr ou à l'ADN codant pour l'ARNr de *Mycoplasma pneumoniae* ou de *Mycoplasma genitalium,* ou des deux, s'ils sont présents dans l'échantillon, pour former des complexes d'acides nucléiques, dans des conditions qui ne permettent pas à ladite sonde de former des complexes d'acides nucléiques hybridés stables avec des bactéries non *Mycoplasma;* et
c) détecter lesdits complexes d'acides nucléiques comme une indication de la présence soit de *Mycoplasma pneumoniae,* soit de *Mycoplasma genitalium,* soit des deux, dans l'échantillon.

14. Procédé selon la revendication 13, dans lequel ladite sonde d'acides nucléiques de ladite étape de mise en contact est complémentaire ou homologue à une séquence d'une sonde quelconque parmi la Sonde 2167, la Sonde 2295, la Sonde 2289, la Sonde 2296, la Sonde 2298, la Sonde 2299, la Sonde 2300, la Sonde 2294, la Sonde 2202, la Sonde 2166, la Sonde 2225, la Sonde 2230 ,ou la Sonde 2297.

15. Procédé pour détecter la présence soit de *Mycoplasma pneumoniae,* soit de *Mycoplasma genitalium,* soit des deux à la fois dans un échantillon, comprenant les étapes qui consistent à:
a) mettre ledit échantillon au contact d'un ensemble d'au moins deux sondes d'acides nucléiques selon l'une quelconque des revendications 9 ou 11;
b) imposer des conditions d'hybridation sur l'échantillon et lesdites sondes d'acides nucléiques pour permettre aux dites sondes de s'hybrider de façon stable à l'ARNr ou à l'ADN codant pour l'ARNr de *Mycoplasma pneumoniae* ou de *Mycoplasma genitalium,* ou des deux, s'ils sont présents dans l'échantillon, pour former des complexes d'acides nucléiques, dans des conditions qui ne permettent pas aux dites sondes de former des complexes d'acides nucléiques hybridés stables avec des bactéries non *Mycoplasma;* et
c) détecter lesdits complexes d'acides nucléiques comme une indication de la présence soit de *Mycoplasma pneumoniae,* soit de *Mycoplasma genitalium,* soit des deux, dans l'échantillon.

16. Procédé selon la revendication 15, dans lequel ledit ensemble de sondes d'acides nucléiques est choisi dans le groupe constitué par les ensembles de sondes
Sonde 2196 et Sonde 2167, Sonde 2162 et Sonde 2202, Sonde 2224 et Sonde 2166, Sonde 2162 et Sonde 2230, Sonde 2167 et Sonde 2219, Sonde 2192 et Sonde 2202, Sonde 2296 et Sonde 2297, ou Sonde 2299 et Sonde 2300.

17. Trousse pour détecter soit Mycoplasma pneumoniae, soit Mycoplasma genitalium, soit les deux, associés à la pneumonie atypique primitive humaine, comprenant une sonde d'acides nucléiques telle que définie dans les revendications 1 à 8 ou un ensemble de sondes d'acides nucléiques tel que défini dans les revendications 9 à 12.
